# EUROPÄISCHE PATENTSCHRIFT

(11) **EP 3 340 940 B1**
(45) Veröffentlichungstag und Bekanntmachung des Hinweises auf die Patenterteilung: **08.05.2019**
(21) Anmeldenummer: 16753926.1
(22) Anmeldetag: 19.08.2016
(51) Int. Cl.: A61F 2/60, A61F 2/64, A61F 2/50, A61F 2/68, A61F 2/74, A61F 2/66, A61F 5/01

(54) **KÜNSTLICHES GELENK**
ARTIFICIAL JOINT
ARTICULATION ARTIFICIELLE

(30) Priorität: 24.08.2015 DE 102015113977
(43) Veröffentlichungstag der Anmeldung: 04.07.2018
(73) Patentinhaber: Otto Bock Healthcare Products GmbH, 1110 Wien (AT)
(72) Erfinder: DIETL, Hans, 3003 Gablitz (AT)
(74) Vertreter: Gramm, Lins & Partner Patent- und Rechtsanwälte PartGmbB
(86) Internationale Anmeldenummer: PCT/EP2016/069724
(87) Internationale Veröffentlichungsnummer: WO 2017/032717

(56) Entgegenhaltungen:
- EP-A1- 2 316 390
- WO-A2-2009/140956
- US-A1- 2005 059 908
- US-A1- 2013 245 524

## Beschreibung

Die Erfindung betrifft ein künstliches Gelenk für untere Extremitäten mit einem Oberteil, das Einrichtungen zur Festlegung an einem Nutzer aufweist, und einem um eine Gelenkachse schwenkbar daran gelagerten Unterteil, das aus einer extendierten Stellung während des Stehens in eine flektierte Stellung einbeugbar ist. Das künstliche Gelenk kann als Hüftgelenk und Knöchelgelenk ausgebildet sein, in einer bevorzugten Ausführungsform ist das künstliche Gelenk als ein Kniegelenk ausgebildet.

Künstliche Gelenke für untere Extremitäten können in Orthesen oder Prothesen eingesetzt werden. Orthesen werden benötigt, um die Funktion der noch vorhandenen unteren Extremität zu unterstützen oder aufrecht zu erhalten. Dazu werden die Orthesen an der noch vorhandenen Extremität festgelegt. Bei einer Knieorthese werden an dem Oberschenkel und an dem Unterschenkel Schienen angelegt, die über ein Orthesenkniegelenk miteinander verbunden sind. Bei einer Knöchelorthese wird der Fuß auf einem Fußteil fixiert, das über ein Orthesenknöchelgelenk mit einer Unterschenkelschiene verbunden ist. Gleiches gilt für eine Hüftgelenkorthese. Grundsätzlich ist es auch möglich, eine Orthese mit mehr als zwei Gelenken vorzusehen.

Fehlende Gliedmaßen werden durch Prothesen ersetzt. Bei Prothesenkniegelenken wird das fehlende Kniegelenk durch ein Prothesenkniegelenk ersetzt, dessen Oberteil an einem Oberschenkelstumpf über einen Oberschenkelschaft festgelegt ist. An dem Oberteil ist das Unterteil des Prothesenkniegelenkes schwenkbar befestigt. An dem Unterteil sind ein Unterschenkelrohr und ein Prothesenfuß, gegebenenfalls mit einem Prothesenknöchelgelenk, angeordnet. Ebenfalls ist es möglich, bei einer Hüftexartikulation das fehlende Hüftgelenk durch eine entsprechende Exoprothese zu ersetzen.

Patienten mit eingeschränkten motorischen Fähigkeiten bedürfen mitunter einer Unterstützung bei kraftaufwendigen Bewegungen. Dazu zählen insbesondere das Aufstehen oder Hinsetzen, da bei diesen Tätigkeiten ein überwiegender Teil des Körpergewichts vertikal bewegt werden muss. Beim Aufstehen muss das Körpergewicht angehoben, beim Hinsetzen kontrolliert abgesenkt werden. Bei Prothesenträgern muss dazu in der Regel das unversorgte Bein die gesamte Arbeit übernehmen, gegebenenfalls unterstützt durch die Arme, bei Orthesenträgern mit nur eingeschränkten motorischen Fähigkeiten im versorgten Bein ist die Situation vergleichbar.

Die US 2013/245524 A1 betrifft eine Knie-Knöchel-Fuß-Orthese mit einem Oberteil, das Einrichtungen zum Festlegen an einem Patienten aufweist, und einem Unterteil, das schwenkbar um eine Gelenkachse an dem Oberteil gelagert ist. Eine Gasdruckfeder als Kraftspeicher ist dem Gelenk zugeordnet. Die Gasdruckfeder wird durch eine Flexionsbewegung aufgeladen und kann eine Extensionsbewegung unterstützen.

Aufgabe der vorliegenden Erfindung ist es, ein künstliches Gelenk bereitzustellen, das eine Hinsetzhilfe und eine Aufstehhilfe bereitstellt, ohne dass in der sitzenden Stellung eine Einschränkung durch eine ungewollte Kraftaufbringung auf das künstliche Gelenk erfolgt.

Erfindungsgemäß wird diese Aufgabe durch ein künstliches Gelenk mit den Merkmalen des Hauptanspruches gelöst. Vorteilhafte Ausgestaltungen und Weiterbildungen der Erfindung sind in den Unteransprüchen, der Beschreibung und den Figuren offenbart.

Das erfindungsgemäße künstliche Gelenk für untere Extremitäten mit einem Oberteil, das Einrichtungen zur Festlegung an einem Nutzer aufweist, und einem um eine Gelenkachse schwenkbar daran gelagerten Unterteil, das aus einer extendierten Stellung, insbesondere während des Stehens, in eine flektierte Stellung einbeugbar ist, sieht vor, dass dem Gelenk ein Kraftspeicher zugeordnet ist, der über eine Kraftübertragungseinrichtung durch eine Flexionsbewegung des Oberteils zu dem Unterteil aufgeladen wird und eine Extensionsbewegung des Unterteils zum Oberteil zumindest über einen Teil der Extensionsbewegung unterstützt, wobei die Kraftübertragungseinrichtung bei einem Beugewinkel zwischen 45° bis 80° ein maximales Extensionsmoment ausübt. In dem extendierten Zustand des Gelenkes ist der Kraftspeicher entspannt oder nur gering vorgespannt. Während der Flexionsbewegung wird eine Kraftübertragungseinrichtung aktiviert, über die der Kraftspeicher aufgeladen wird. Durch das Aufladen wird beispielsweise beim Hinsetzen eine Unterstützung des unversorgten Beines erreicht, da zumindest ein Teil derjenigen Energie, die aufgewendet werden müsste, um das Absenken des Körpers aufzuhalten, durch das Aufladen des Kraftspeichers aufgebracht wird. Bei einer umgekehrten Bewegung, also bei der Extension, wird die Aufstehbewegung durch das Entladen des Kraftspeichers unterstützt. Die Kraftübertragungseinrichtung ist dabei so ausgebildet, dass in demjenigen Bereich des Gelenkwinkels, in dem für den Patienten ein maximaler Kraftaufwand notwendig wäre, nämlich bei einem Beugewinkel zwischen 45° und 80°, ein maximales Extensionsmoment ausgeübt wird, so dass beim Aufstehen eine maximale Unterstützung bzw. beim Hinsetzen eine maximale Dämpfung oder gegenwirkende Kraft aufgebracht wird. Sitzt der Patient oder Nutzer, ist in der Regel das Gelenk in einem Winkel eingebeugt, der größer als 80° ist, beispielsweise 90°, so dass das maximale Unterstützungsmoment nur in dem Winkelbereich aufgebracht wird, in dem tatsächlich eine Bewegung stattfindet. Bei einem Winkel von ungefähr 80° wird die Hinsetzbewegung gerade abgeschlossen oder die Aufstehbewegung gerade begonnen. Durch die Ausgestaltung des Gelenkes mit einem Kraftspeicher ist es nicht notwendig, motorische Antriebe vorzusehen, vielmehr kann eine einfache Umwandlung und Rückumwandlung der jeweils auftretenden Energien stattfinden. Das Unterstützungsmoment ist einstellbar und wird vorteilhafterweise auf einem Niveau gehalten, dass unterhalb des benötigten Momentes für die beabsichtigte Bewegung, z.B. Aufstehen oder Hinsetzen, liegt, um dem Nutzer die Kontrolle über die Bewegung zu belassen.

Das Gelenk ist vorteilhafterweise als Orthesen- oder Prothesengelenk ausgebildet und kann insbesondere als Kniegelenk oder Hüftgelenk ausgebildet sein.

Über den gesamten Flexionsbereich des künstlichen Gelenkes kann ein Extensionsmoment anliegen, so dass bereits zu Beginn der Flexion ein entgegenwirkendes Moment auftritt, wenn eine Einbeugung stattfindet. Dies bedeutet, dass ein Einbeugen des betreffenden Gelenkes erst gegen einen Anfangswiderstand möglich ist. Dies führt dazu, dass der Patient in der extendierten Stellung, beispielsweise im Stehen, eine erhöhte Stabilität und Sicherheit erhält.

Vorteilhafterweise ist die Kraftübertragungseinrichtung so ausgebildet, dass bei einem Beugewinkel von über 90° kein Extensionsmoment anliegt, also nach dem Extensionsmoment-Maximum ein Abfall des Extensionsmomentes auf Null oder nahezu Null vorgesehen ist.

Der Kraftspeicher kann als Feder, Federpaket, hydraulischer Kraftspeicher oder pneumatischer Kraftspeicher ausgebildet sein, was eine Umwandlung in eine andere Energieform, beispielsweise elektrische Energie, unnötig macht. Die in der Feder oder im Federpaket bzw. in den Druckspeichern gespeicherte Energie kann unmittelbar zum Antrieb bzw. Ausüben des Extensionsmomentes eingesetzt werden, ohne dass es weiterer Komponenten bedarf.

Der Kraftübertragungseinrichtung kann eine Sperr- oder Ausrückeinrichtung zugeordnet ist, die in Abhängigkeit von der Belastung oder Stellung des künstlichen Gelenkes das Gelenk sperrt oder eine Kraftübertragung unterbricht. Eine permanente Unterstützung der Extensionsbewegung kann unerwünscht sein, beispielsweise bei einem entlasteten Unterteil, einer sitzenden Position oder einer glatten Oberfläche. Daher sind Sperr- oder Ausrückeinrichtungen vorteilhaft, über die eine Sperrung des Gelenkes oder der Kraftübertragung oder eine Unterbrechung der Kraftübertragung in Abhängigkeit von Einsatzbedingungen des künstlichen Gelenkes erreicht werden kann. Die Sperrung des Gelenkes kann über eine formschlüssige Sperrung der Bewegung des Oberteils zu dem Unterteil erfolgen, bei der ein Sperrelement in eine Ausnehmung eingreift.

Die Kraftübertragungseinrichtung kann auch nur über einen definierten Beugewinkelbereich wirksam sein, wobei die Sperr- oder Ausrückeinrichtung bei Erreichen eines Beugewinkels die Kraftübertragung unterbricht oder bei weiterhin vorhandener Kraftaufbringung eine Bewegung des künstlichen Gelenkes sperrt. Eine solche Ausrückeinrichtung kann beispielsweise als Ausrückkupplung ausgebildet sein. Derjenige Winkel, bei dem ein Ausrücken erfolgt, kann einstellbar sein, ebenso kann das Kraftniveau des Kraftspeichers einstellbar sein, um eine Anpassung an unterschiedliche Patienten mit unterschiedlichen Gewichten oder unterschiedlichen Leistungsfähigkeiten vornehmen zu können. Durch das Ausrücken der Kraftübertragungseinrichtung wird das Gelenk freigeschaltet und eine freie Beweglichkeit ermöglicht, so dass beispielsweise bei einem Kniegelenk die beim Sitzen weiterhin ausgeführten kleinen Bewegungen mehr oder weniger ohne Widerstand ausgeführt werden können. Auch ist es möglich, dass das Gelenk gesperrt wird, zumindest in Extensionsrichtung, um bei einer definierten Position und oder Belastungssituation keine Streckung zu erlauben.

Eine Weiterbildung der Erfindung sieht vor, dass der Sperr- oder Ausrückeinrichtung ein Aktuator zugeordnet ist, der die Sperr- oder Ausrückeinrichtung bei Vorliegen oder Überschreiten einer auf das Unterteil einwirkenden Axialkraft deaktiviert. Durch den belastungsabhängig arbeitenden Aktuator wird sichergestellt, dass eine Extensionsunterstützung beispielsweise des Hüft- oder Kniegelenkes erst dann vorgenommen wird, wenn der Nutzer des künstlichen Gelenkes eine ausreichend hohe Axialkraft auf das Unterteil, insbesondere den Fuß aufbringt, um so ein Wegrutschen des Fußteils oder des Prothesenfußes zu vermeiden. Der Aktuator ist in vorteilhafter Weise mechanisch ausgebildet und wird durch die Axialkraft selbst aktuiert. Der Aktuator kann ein Schieber, eine Druckstange oder auch ein Kolben einer Hydraulikeinheit sein, über die die Axialkraft zu der Sperr- oder Ausrückeinheit geleitet wird. Die Hydraulikeinheit ermöglicht eine nahezu beliebige Kraftführung aufgrund der Hydraulikleitungen und zudem eine Kraft- und/oder Wegübersetzung, wodurch sich der Gestaltungsspielraum für das künstliche Gelenk erhöht.

Das künstliche Gelenk kann in einer Ausführungsform als entriegelbares und verriegelbares Sperrkniegelenk ausgebildet sein, also eine Verriegelungseinrichtung aufweisen, die in der Regel eine formschlüssige Verriegelung des Gelenkes gegen eine Flexion ausübt. Die Verriegelungseinrichtung kann manuell oder motorisch entriegelbar sein, um überhaupt eine Flexion zu ermöglichen.

In einer Ausführungsform der Erfindung ist es vorgesehen, dass mehrere Kraftspeicher parallel oder in Reihe geschaltet angeordnet sind, um den stets geringen Bauraum möglichst optimal auszunutzen, eine modulare Fertigung zu ermöglichen und eine individuelle Anpassung an den jeweiligen Patienten durchzuführen und ein Entfernen oder Hinzufügen von Kraftspeichern zu ermöglichen.

Zwischen dem Gelenk und dem Kraftspeicher kann ein Getriebe angeordnet sein, um eine Kraft-Weg-Übersetzung bereitzustellen.

Das Getriebe kann als Kurvenscheibengetriebe, Hebelgetriebe, Hydraulikgetriebe oder Pneumatikgetriebe ausgebildet sein.

Die Kraftausübungsrichtung der Kraftübertragungseinrichtung kann in der Stellung des Gelenkes, in der das maximale Extensionsmoment anliegt, vorteilhafterweise senkrecht zur Längserstreckung des Oberteils oder des Unterteils verlaufen, um eine optimale Ausnutzung der in dem Kraftspeicher vorhandenen Energie zu ermöglichen.

Die Kraftübertragungseinrichtung kann als Druckstange oder flexibles, unelastisches Zugmittel ausgebildet sein, das über Rollen, Zapfen oder andere Umlenkeinrichtungen geführt ist, um eine an den Bauraum angepasste und optimierte Kraftführung und Kraftumleitung zu erreichen.

Eine Weiterbildung der Erfindung sieht einen elastischen Streckanschlag vor, der dem künstlichen Gelenk zugeordnet ist. Der Endpunkt des Streckanschlages kann einstellbar sein, so dass die maximale Streckposition des Gelenkes individuell an den Nutzer angepasst werden kann. Über einen festgelegten Winkelbereich vor Erreichen der maximalen Streckung wird über eine elastische Komponente innerhalb des Streckanschlages die Extensionsbewegung abgebremst, wodurch eine Verringerung des in Richtung der Extension wirkenden Momentes erreicht wird. Dadurch wird unter anderem verhindert, dass die Extensionsbewegung schlagartig bei Erreichen der maximalen Streckung aufhört, was eine unnatürliche Bewegung darstellen würde.

Nachfolgend wird ein Ausführungsbeispiel der Erfindung anhand der beigefügten Figuren näher erläutert. Es zeigen:
- Figur 1: - ein Prothesenkniegelenk in extendierter Stellung;
- Figur 2: - ein Gelenk gemäß Fig. 1 in eingebeugter Stellung; sowie
- Figur 3: - eine schematische Darstellung einer Funktionskette.

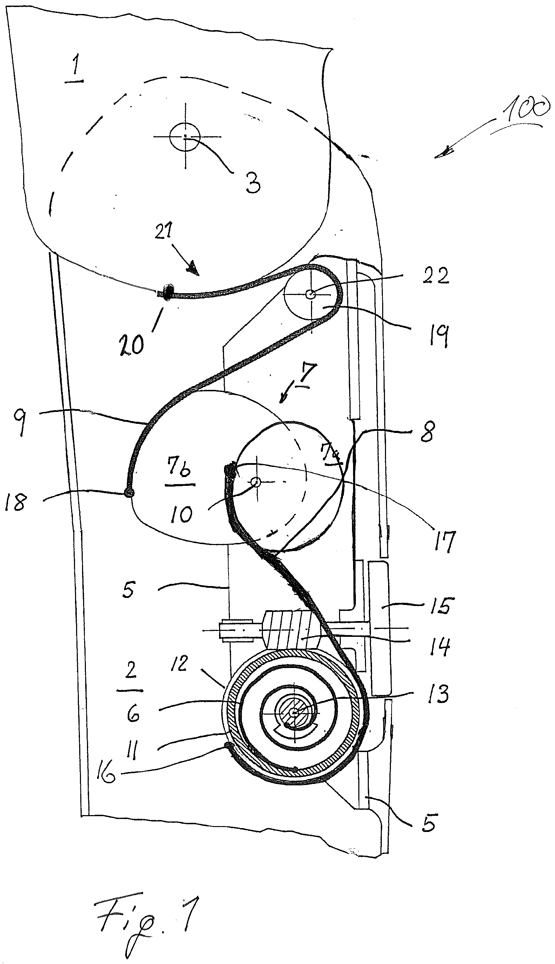

Figur 1 zeigt ein künstliches Gelenk 100 für untere Extremitäten mit einem Oberteil 1, das an einem Unterteil 2 gelenkig um eine Schwenkachse 3 gelagert ist. An dem Oberteil 1 sind Einrichtungen zur Festlegung an einem Nutzer oder Patienten angeordnet oder ausgebildet, deren Ausgestaltung von der Art des Gelenks 100 abhängt. Sofern das künstliche Gelenk 100 als ein Orthesengelenk ausgebildet ist, sind das Oberteil 1 und das Unterteil 2 als eine Schienenanordnung ausgebildet, die über Gurte, Manschetten oder andere, das zu unterstützende Körperglied umschlingende Befestigungsmittel fixiert. Ist das Gelenk als Knöchelgelenk ausgebildet, erfolgt die Fixierung an dem Fuß und dem Unterschenkel, bei einer Ausgestaltung des künstlichen Gelenkes als ein Orthesenkniegelenk erfolgt die Festlegung zumindest an dem Unterschenkel und dem Oberschenkel, bei der Ausgestaltung des künstlichen Gelenks als Hüftgelenk ist die Orthese an dem Rumpf und dem Oberschenkel festgelegt. Bei einer Ausgestaltung des künstlichen Gelenks 100 als Prothese wird das Oberteil 1 über einen Adapter und einen Schaft an der jeweiligen verbliebenen Gliedmaße fixiert, beispielsweise über einen Unterschenkelschaft an einem Unterschenkel, einem Oberschenkelschaft an einem Oberschenkel bei einer Ausgestaltung des künstlichen Gelenks als ein Prothesenknielenk oder an dem Rumpf bei einer Ausgestaltung des künstlichen Gelenkes als Hüftgelenk. Das jeweilige Unterteil ist dann als Prothesenfuß, Unterschenkelrohr oder Oberschenkelrohr ausgebildet.

In der Ausführungsform gemäß Figur 1 ist das künstliche Gelenk als ein Prothesenkniegelenk ausgebildet, bei dem im Unterteil 2 ein Kraftspeicher 6 in Gestalt einer Spiralfeder angeordnet, die über Kraftübertragung in Gestalt von Riemen oder Gurten 8, 9 und ein Getriebe 7 in Gestalt eines Kurvenscheibengetriebes mit dem Oberteil 1 gekoppelt ist. Der dem Oberteil 1 zugeordnete Riemen 9 ist flexibel und unelastisch ausgebildet und in der dargestellten maximal extendierten Stellung des Prothesenkniegelenkes an einem ersten Befestigungspunkt 20 befestigt. Der Riemen 9 liegt an der im wesentlichen kreissegmentförmig ausgebildeten Kontur 21 des distalen Endes des Oberteils 1 an und wird von dort über eine Umlenkrolle 19, die innerhalb des Unterteils 2 in einem separaten Modulgehäuse 5 um eine Drehachse 22 drehbar gelagert ist, umgelenkt und mit seinem zweiten Ende an dem Kurvenscheibengetriebe 7 an einem zweiten Befestigungspunkt 18 gelagert. Das Kurvenscheibengetriebe 7 besteht aus zwei Kurvenscheiben 7a, 7b, die um eine gemeinsame Schwenkachse 10 drehbar in dem Gehäuse 5 gelagert sind. Der zweite Riemen 8 ist an der ersten Kurvenscheibe 7a an einem ersten Befestigungspunkt 17 gelagert, das gegenüberliegende Ende des zweiten Riemens 8 ist an einem zweiten Befestigungspunkt 16 an einer Abtriebsscheibe 12 befestigt, die mit einem Federgehäuse 11 für die Spiralfeder 6 gekoppelt ist. Die Abtriebsscheibe 12 ist schwenkbar um eine Drehachse 13 in dem Gehäuse 5 gelagert. Alle Dreh- und Schwenkachsen 10, 13, 22 sind parallel zueinander orientiert und verlaufen im Wesentlichen parallel zu der Schwenkachse 3 des künstlichen Gelenks.

Ebenfalls an dem Gehäuse 5 ist ein Schneckenrad 14 gelagert, das über eine manuell betätigbare Drehscheibe 15 verdreht werden kann. Das Schneckenrad 14 kämmt mit einem Gewinde, das auf dem Außenumfang des Federgehäuses 11 ausgebildet ist, so dass die Vorspannung der Spiralfeder 6 eingestellt werden kann. Über die Vorspannung der Spiralfeder 6 ist es möglich, die aufzubringende Zugkraft über die Riemen 8, 9 und damit ein Extensionsmoment einzustellen, das auch als permanentes Streckmoment wirkt und gegen ein Einbeugen des künstlichen Gelenkes 100 gerichtet ist. Wird die Vorspannung der Spiralfeder 6 groß genug eingestellt, ergibt sich ein Grundwiderstand gegen ein Einbeugen und somit ein vergrößertes Sicherheitsgefühl des Nutzers des künstlichen Gelenkes. Beide Riemen 8, 9 sind an dem äußeren Umfang der jeweiligen Kurvenscheibe 7a, 7b fixiert. Die Kurvenscheiben weisen eine exzentrische Kontur um die Drehachse 10 auf, wobei die Konturen beider Kurvenscheiben 7a, 7b voneinander verschieden sind, so dass sich bei einer nahezu gleichbleibenden Rückstellkraft aufgrund der Spannung innerhalb der Spiralfeder 6 ein über den Beugewinkel des Oberteils 1 relativ zu dem Unterteil 2 veränderndes Extensionsmoment ergibt. Das Extensionsmoment ist abhängig von den jeweiligen Hebelverhältnissen und Abständen der Riemen 8, 9 von der Schwenkachse 10 sowie dem ersten Befestigungspunkt 20 des Riemens 9 an dem Oberteil 1 und der Position der Umlenkrolle 19. Das Getriebe 7 ist in Verbindung mit der Spiralfeder 6 so ausgelegt, dass der Verlauf des um die Schwenkachse 3 aufgebrachten Drehmomentes in Abhängigkeit von dem Beugewinkel des Oberteils 1 relativ zu dem Unterteil 2 einen Maximalwert in einem Beugebereich zwischen 45° und 80° aufweist und bei einer weiteren Einbeugung über diesen Zielwinkelbereich hinaus stark abfällt. Ein der Einbeugung entgegenwirkendes Drehmoment wird in einem ersten Winkelbereich zwischen einer vollen Kniestreckung und einer Einbeugung bis ungefähr 20° auf 20% der maximal möglichen Drehmomentunterstützung begrenzt. Ab Erreichen eines Beugewinkels von ca. 25° steigt das Widerstandsmoment stark an und erreicht im Bereich von ca. 80° Gelenkwinkel sein Maximum, wobei in dem Bereich zwischen 45° und 80° ein nahezu gleichbleibendes Momentenniveau anliegt. Nach Erreichen des Gelenkwinkels von 0° fällt das Extensionsmoment steil ab, vorzugsweise wird dann der Kraftspeicher 6 in Gestalt der Spiralfeder von der Kraftübertragungseinrichtung 8, 9 getrennt oder das Kurvenscheibengetriebe 7 ist so ausgelegt, dass ab einer Winkelstellung von 90° nur noch ein minimales Streckmoment aufgebracht wird.

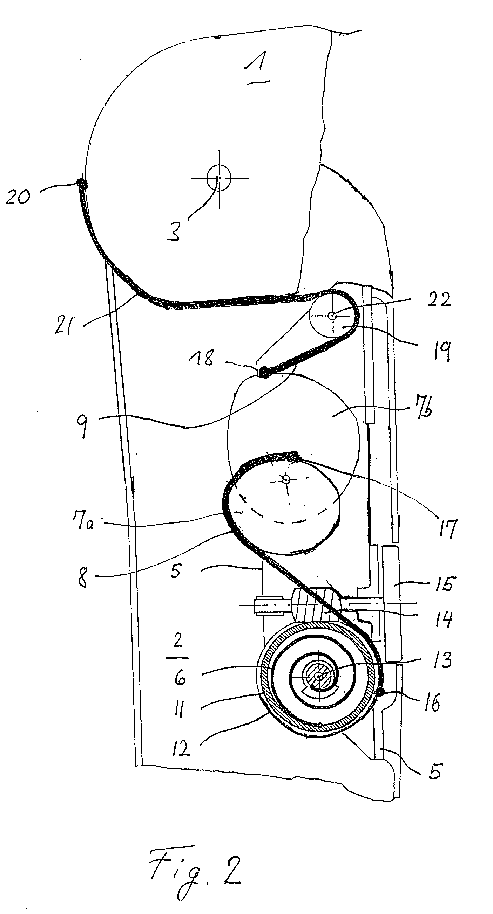

In Figur 2 ist das künstliche Gelenk in einer eingebeugten Stellung dargestellt, das Oberteil 1 ist in einem nahezu rechten Winkel zu dem Unterteil 2 um die Schwenkachse 3 verschwenkt. Das Kurvenscheibengetriebe 7 wurde im Uhrzeigersinn verschwenkt, die Spiralfeder 6 wurde über die Antriebsscheibe 2 entgegen dem Uhrzeigersinn aufgewickelt und aufgeladen. In der dargestellten 90°-Stellung kann über eine Ausrückkopplung eine Kraftübertragung zwischen dem Kraftspeicher in Gestalt der Spiralfeder 6 und der Kraftübertragungseinrichtungen 8, 9 unterbrochen sein, so dass in dieser Stellung und bei einer weiteren Einbeugung kein Extensionsmoment, also ein die Streckung des Oberteils 1 relativ zu dem Unterteil 2 bewirkende Kraft aufgebracht wird. Wird dann eine Streckung initiiert, greift eine Kraftübertragung ab einem vorbestimmten Winkel, beispielsweise bei einem Gelenkwinkel von 80°, wieder ein und der Kraftspeicher 6 unterstützt die Extensionsbewegung mit einem maximalen Extensionsmoment in einem Winkelbereich zwischen 45° und 80°. Bei einer weiteren Streckung, also bei einer Verringerung des Gelenkwinkels, verringert sich das Extensionsmoment aufgrund der Geometrien der Kurvenscheiben 7a, 7b des Kurvengetriebes 7 bis auf einen vorgegebenen Wert, der variabel einstellbar ist. Die Einstellung erfolgt über das Drehrad 15 und die Vorspannung über das Schneckengetriebe 14 und die Vorspannung der Spiralfeder 6.

Die Gelenkeinrichtung kann auch als Sperrgelenk mit einer separaten, formschlüssigen Sperre ausgebildet sein, so dass erst nach Lösen der Sperre überhaupt eine Extensionsbewegung, also eine Einbeugung stattfinden kann.

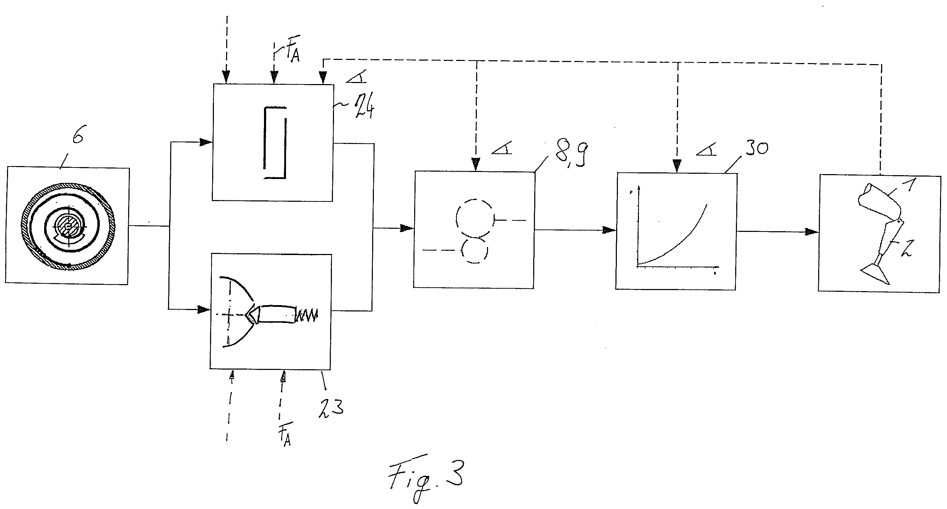

Figur 3 zeigt eine schematische Darstellung einer Funktionskette einzelner Komponenten des künstlichen Gelenks. Ein Energie- oder Kraftspeicher 6, beispielsweise in Gestalt einer Spiralfeder, eines pneumatischen oder hydraulischen Druckspeichers, eines anderen Federspeichers oder dergleichen, ist über eine Sperreinrichtung 23 oder über eine Ausrückeinrichtung 24 mit zumindest einer Kraftübertragungseinrichtung 8, 9 verbunden. Die Kraftübertragungseinrichtung 8, 9 ist mit einem progressiven, elastischen Gelenkanschlag 30 verbunden, der insbesondere als Extensionsanschlag ausgebildet ist, um ein abruptes Anschlagen des Unterteils 2 an dem Oberteil 1 in der Extensionsstellung zu vermeiden. Über einen definierten Winkelbereich vor der vollständigen Streckung wird der Widerstand der Extension erhöht, vorteilhafterweise progressiv erhöht, so dass eine unkontrollierte Extension vermieden wird.

Der Kraftspeicher 6 kann gemäß der Ausgestaltung der Figuren 1 und 2 ausgebildet sein. Die Sperreinrichtung 23 ebenso wie die Ausrückeinrichtung 24 bzw. Kupplung kann in Abhängigkeit von einer Axialkraft FA geschaltet werden, die in bzw. auf das Unterteil 2 wirkt. Um zu verhindern, dass bei einer zu geringen Axialkraft auf das Unterteil 2 bzw. auf den Prothesenfuß das Unterteil 2 durch den Kraftspeicher 6 in die Extensionsstellung vorbewegt wird, wird die Energieübertragung von dem Kraftspeicher 6 über die Kraftübertragungseinrichtung 8, 9 auf das Oberteil 1 bzw. Unterteil 2 erst dann ermöglicht, wenn das Fußteil ein ausreichendes Widerlager bildet, also der Nutzer des künstlichen Gelenkes steht oder mit einer ausreichenden Belastung sitzt. Bei einem unbelasteten Sitzen wirkt keine oder nahezu keine Axialkraft in dem Unterteil 2, so dass eine Kraftübertragung von dem Energiespeicher zur Extension des Gelenkes gesperrt wird. Alternativ zu einer Sperrung durch die Sperreinrichtung 23 kann dies auch durch die Ausrückeinrichtung 24 bzw. eine schaltbare Kupplung erfolgen. Wird eine ausreichend hohe Axialkraft innerhalb des Unterteils 2 detektiert, kann die Situation angenommen werden, dass der Patient aufstehen möchte oder steht, so dass eine Aufstehunterstützung oder Extensionsunterstützung sinnvoll ist.

Alternativ oder ergänzend zu der Berücksichtigung der Axialkraft FA kann eine Sperre 23 bzw. eine Ausrückvorrichtung 24 bzw. Kupplung individuell durch ein Signal des Nutzers aktiviert bzw. deaktiviert werden, was durch die Pfeile angedeutet ist. Die Aktivierung kann mechanisch oder über Betätigung eines Schalters elektronisch erfolgen. Die Sperre 23 und die Ausrückeinrichtung 24 sind somit als schaltbare Komponenten ausgebildet, die entweder automatisch in Abhängigkeit von dem Erreichen eines Grenzwertes, beispielsweise der Axialkraft oder individuell durch den Nutzer geschaltet werden kann.

Sowohl die Ausrückvorrichtung 24 als auch die Sperre 23 sind in Abhängigkeit von dem Genlenkwinkel schaltbar oder steuerbar. So kann die Kraft über die Ausrückvorrichtung über einen bestimmten Winkelbereich vollständig über die Ausrückvorrichtung übertragen werden bzw. kann die Sperre 23 über einen bestimmten Winkelbereich deaktiviert oder aktiviert werden. Beispielsweise kann im Endbereich der Extension eine verringerte Kraftübertragung aus dem Energiespeicher 6 auf das künstliche Gelenk erfolgen, um einen zu harten Anschlag in die maximale Streckung zu vermeiden. Ebenfalls kann winkelabhängig die Kraftübertragungseinrichtung 8, 9 wirksam werden, was durch eine spezielle Anordnung und gegebenenfalls Verstellbarkeit der jeweiligen Komponenten der Kraftübertragungseinrichtung gewährleistet sein kann. Der Anschlag 30 kann über den Gelenkwinkel verstellt werden, je größer der Gelenkwinkel ist, also der zwischen der Längserstreckung des Oberteils und der Längserstreckung des Unterteils eingeschlossene Winkel, desto größer kann der Widerstand in dem elastischen Anschlag ausgebildet werden.

## Patentansprüche

1. Künstliches Gelenk für untere Extremitäten mit einem Oberteil (1), das Einrichtungen zur Festlegung an einem Nutzer aufweist, und einem um eine Gelenkachse (3) verschwenkbar daran gelagerten Unterteil (2), das aus einer extendierten Stellung in eine flektierte Stellung einbeugbar ist, wobei dem Gelenk (100) ein Kraftspeicher (6) zugeordnet ist, der über eine Kraftübertragungseinrichtung (8, 9) durch eine Flexionsbewegung des Oberteils (1) zu dem Unterteil (2) aufgeladen wird und eine Extensionsbewegung des Unterteils (2) zu dem Oberteil (1) zumindest über einen Teil der Extensionsbewegung unterstützt, **dadurch gekennzeichnet, dass** die Kraftübertragungseinrichtung (8, 9) bei einem Beugewinkel zwischen 45° bis 80° ein maximales Extensionsmoment ausübt.

2. Künstliches Gelenk nach Anspruch 1, **dadurch gekennzeichnet, dass** das Gelenk (100) als Orthesen- oder Prothesengelenk ausgebildet ist.

3. Künstliches Gelenk nach Anspruch 1 oder 2, **dadurch gekennzeichnet, dass** das Gelenk (100) als Kniegelenk oder Hüftgelenk ausgebildet ist.

4. Künstliches Gelenk nach einem der voranstehenden Ansprüche, **dadurch gekennzeichnet, dass** über den gesamten Flexionsbereich ein Extensionsmoment anliegt.

5. Künstliches Gelenk nach einem der Ansprüche 1 bis 3, **dadurch gekennzeichnet, dass** bei einem Beugewinkel über 90° kein Extensionsmoment anliegt.

6. Künstliches Gelenk nach einem der voranstehenden Ansprüche, **dadurch gekennzeichnet, dass** der Kraftspeicher (6) als Feder, Federpaket, hydraulischer Kraftspeicher oder pneumatischer Kraftspeicher ausgebildet ist.

7. Künstliches Gelenk nach einem der voranstehenden Ansprüche, **dadurch gekennzeichnet, dass** der Kraftübertragungseinrichtung (8, 9) eine Sperr- oder Ausrückeinrichtung (23, 24) zugeordnet ist, die in Abhängigkeit von der Belastung oder Stellung des künstlichen Gelenkes (100) eine Extension sperrt oder Kraftübertragung unterbricht.

8. Künstliches Gelenk nach Anspruch 7, **dadurch gekennzeichnet, dass** die Kraftübertragungseinrichtung (8, 9) nur über einen definierten Beugewinkelbereich wirksam ist und die Sperr- oder Ausrückeinrichtung (23, 24) bei Erreichen eines definierten Beugewinkels das künstliche Gelenk (100) sperrt oder die Kraftübertragung unterbricht.

9. Künstliches Gelenk nach Anspruch 7 oder 8, **dadurch gekennzeichnet, dass** der Sperr- oder Ausrückeinrichtung (23, 24) ein Aktuator zugeordnet ist, der die Sperr- oder Ausrückeinrichtung (23, 24) bei Vorliegen oder Überschreiten einer auf das Unterteil (2) einwirkenden Axialkraft (F_{A}) deaktiviert.

10. Künstliches Gelenk nach einem der voranstehenden Ansprüche, **dadurch gekennzeichnet, dass** die Ausrückeinrichtung (24) als schaltbare Kupplung ausgebildet ist.

11. Künstliches Gelenk nach einem der voranstehenden Ansprüche, **dadurch gekennzeichnet, dass** das Kraftniveau des Kraftspeichers (6) einstellbar ist.

12. Künstliches Gelenk nach einem der voranstehenden Ansprüche, **dadurch gekennzeichnet, dass** das Gelenk (100) als entriegelbares und verriegelbares Sperrgelenk ausgebildet ist.

13. Künstliches Gelenk nach einem der voranstehenden Ansprüche, **dadurch gekennzeichnet, dass** mehrere Kraftspeicher (6) parallel oder in Reihe geschaltet angeordnet sind.

14. Künstliches Gelenk nach einem der voranstehenden Ansprüche, **dadurch gekennzeichnet, dass** zwischen dem Gelenk (100) und dem Kraftspeicher (6) ein Getriebe (7) angeordnet ist.

15. Künstliches Gelenk nach Anspruch 14, **dadurch gekennzeichnet, dass** das Getriebe (7) als Kurvenscheibengetriebe (7a, 7b), Hebelgetriebe, Hydraulikgetriebe oder Pneumatikgetriebe ausgebildet ist.

16. Künstliches Gelenk nach einem der voranstehenden Ansprüche, **dadurch gekennzeichnet, dass** die Kraftwirkungsrichtung der Kraftübertragungseinrichtung (8, 9) in der Stellung des Gelenkes (100), in der das maximale Extensionsmoment anliegt, senkrecht zu der Längserstreckung des Oberteils (1) oder des Unterteils (2) ausgerichtet ist.

17. Künstliches Gelenk nach einem der voranstehenden Ansprüche, **dadurch gekennzeichnet, dass** die Kraftübertragungseinrichtung (8, 9) als eine Druckstange oder ein flexibles Zugmittel ausgebildet ist.

18. Künstliches Gelenk nach einem der voranstehenden Ansprüche, **gekennzeichnet durch** einen elastischen Streckanschlag (30), der in einem festgelegten Winkelbereich vor Erreichen der maximalen Streckung die Extensionsbewegung bremst.

## Claims

1. An artificial joint for lower extremities, with an upper part (1), which has mechanisms for securing to a user, and a lower part (2), which is mounted pivotably thereon about a joint axis (3) and which can be bent inward from an extended position to a flexed position, wherein the joint (100) is assigned a force accumulator (6) which is charged via a force transmission mechanism (8, 9) by a flexion movement of the upper part (1) relative to the lower part (2) and supports an extension movement of the lower part (2) relative to the upper part (1) at least over a part of the extension movement, **characterized in that** the force transmission mechanism (8, 9) exerts a maximum moment of extension at a flexion angle between 45° and 80°.

2. The artificial joint as claimed in claim 1, **characterized in that** the joint (100) is designed as an orthotic or prosthetic joint.

3. The artificial joint as claimed in claim 1 or 2, **characterized in that** the joint (100) is designed as a knee joint or hip joint.

4. The artificial joint as claimed in one of the preceding claims, **characterized in that** an extension moment applies over the entire flexion range.

5. The artificial joint as claimed in one of claims 1 through 3, **characterized in that** no extension moment applies at a flexion angle of over 90°.

6. The artificial joint as claimed in one of the preceding claims, **characterized in that** the force accumulator (6) is designed as a spring, spring assembly, hydraulic force accumulator or pneumatic force accumulator.

7. The artificial joint as claimed in one of the preceding claims, **characterized in that** the force transmission mechanism (8, 9) is assigned a blocking or releasing mechanism (23, 24) which, depending on the loading or position of the artificial joint (100), blocks an extension or interrupts force transmission.

8. The artificial joint as claimed in claim 7, **characterized in that** the force transmission mechanism (8, 9) is active only over a defined flexion angle range, and the blocking or releasing mechanism (23, 24) blocks the artificial joint (100) or interrupts the force transmission when a defined flexion angle is reached.

9. The artificial joint as claimed in claim 7 or 8, **characterized in that** the blocking or releasing mechanism (23, 24) is assigned an actuator which deactivates the blocking or releasing mechanism (23, 24) when an axial force (F_{A}) acting on the lower part (2) is present or is exceeded.

10. The artificial joint as claimed in one of the preceding claims, **characterized in that** the releasing mechanism (24) is designed as a switchable coupling.

11. The artificial joint as claimed in one of the preceding claims, **characterized in that** the force level of the force accumulator (6) is adjustable.

12. The artificial joint as claimed in one of the preceding claims, **characterized in that** the joint (100) is designed as an unlockable and lockable blocking joint.

13. The artificial joint as claimed in one of the preceding claims, **characterized in that** several force accumulators (6) are arranged in parallel or in series connection.

14. The artificial joint as claimed in one of the preceding claims, **characterized in that** a gear (7) is arranged between the joint (100) and the force accumulator (6).

15. The artificial joint as claimed in claim 14, **characterized in that** the gear (7) is designed as a cam disk gear (7a, 7b), lever gear, hydraulic gear or pneumatic gear.

16. The artificial joint as claimed in one of the preceding claims, **characterized in that**, in the position of the joint (100) in which the maximum moment of extension applies, the force application direction of the force transmission mechanism (8, 9) is perpendicular to the longitudinal extent of the upper part (1) or of the lower part (2).

17. The artificial joint as claimed in one of the preceding claims, **characterized in that** the force transmission mechanism (8, 9) is designed as a push rod or a flexible pulling means.

18. The artificial joint as claimed in one of the preceding claims, **characterized by** an elastic extension stop (30) which, in a defined angle range, brakes the extension movement before the maximum extension is reached.

## Revendications

1. Articulation artificielle pour des extrémités inférieures, comportant une partie supérieure (1) pourvue de moyens d'immobilisation sur un utilisateur, et une partie inférieure (2) qui est montée sur celle-ci de façon mobile en basculement autour d'un axe d'articulation (3) et qui peut fléchir d'une position en extension jusque dans une position en flexion, un accumulateur de force (6) étant associé à l'articulation (100), qui est chargé par un mouvement de flexion de la partie supérieure (1) par rapport à la partie inférieure (2) via un moyen de transmission de force (8, 9) et qui soutient un mouvement d'extension de la partie inférieure (2) par rapport à la partie supérieure (1) sur une partie au moins du mouvement d'extension, **caractérisée en ce que** le moyen de transmission de force (8, 9) exerce un couple d'extension maximal pour un angle de flexion entre 45° et 80°.

2. Articulation artificielle selon la revendication 1, **caractérisée en ce que** l'articulation (100) est réalisée sous forme d'articulation orthétique ou prothétique.

3. Articulation artificielle selon la revendication 1 ou 2, **caractérisée en ce que** l'articulation (100) est réalisée sous forme d'articulation de genou ou de hanche.

4. Articulation artificielle selon l'une des revendications précédentes, **caractérisée en ce qu'**un couple d'extension s'applique sur toute la plage de flexion.

5. Articulation artificielle selon l'une des revendications 1 à 3, **caractérisée en ce qu'**aucun couple d'extension ne s'applique à un angle de flexion supérieur à 90°.

6. Articulation artificielle selon l'une des revendications précédentes, **caractérisée en ce que** l'accumulateur de force (6) est réalisé sous forme de ressort, d'empilement de ressorts, d'accumulateur de force hydraulique ou d'accumulateur de force pneumatique.

7. Articulation artificielle selon l'une des revendications précédentes, **caractérisée en ce qu'**un moyen de blocage ou de débrayage (23, 24) est associé au moyen de transmission de force (8, 9), qui bloque une extension ou interrompt une transmission de force en fonction de la charge ou de la position de l'articulation artificielle (100).

8. Articulation artificielle selon la revendication 7, **caractérisée en ce que** le moyen de transmission de force (8, 9) n'agit que sur une plage angulaire de flexion définie et le moyen de blocage ou de débrayage (23, 24) bloque l'articulation artificielle (100) ou interrompt la transmission de force lorsqu'un angle de flexion défini est atteint.

9. Articulation artificielle selon la revendication 7 ou 8, **caractérisée en ce qu'**un actionneur est associé au moyen de blocage ou de débrayage (23, 24), qui désactive le moyen de blocage ou de débrayage (23, 24) en présence ou lors d'un dépassement d'une force axiale (F_{A}) agissant sur la partie inférieure (2).

10. Articulation artificielle selon l'une des revendications précédentes, **caractérisée en ce que** le moyen de débrayage (24) est réalisé sous forme de coupleur commutable.

11. Articulation artificielle selon l'une des revendications précédentes, **caractérisée en ce que** le niveau de force de l'accumulateur de force (6) est réglable.

12. Articulation artificielle selon l'une des revendications précédentes, **caractérisée en ce que** l'articulation (100) est réalisée sous forme d'articulation de blocage déverrouillable et verrouillable.

13. Articulation artificielle selon l'une des revendications précédentes, **caractérisée en ce que** plusieurs accumulateurs de force (6) sont agencés en étant branchés en parallèle ou en série.

14. Articulation artificielle selon l'une des revendications précédentes, **caractérisée en ce qu'**un engrenage (7) est agencé entre l'articulation (100) et l'accumulateur de force (6).

15. Articulation artificielle selon la revendication 14, **caractérisée en ce que** l'engrenage (7) est réalisé sous forme d'engrenage à came (7a, 7b), d'engrenage à levier, d'engrenage hydraulique ou d'engrenage pneumatique.

16. Articulation artificielle selon l'une des revendications précédentes, **caractérisée en ce que** la direction d'action de force du moyen de transmission de force (8, 9) est orientée perpendiculairement à l'extension longitudinale de la partie supérieure (1) ou de la partie inférieure (2) dans cette position de l'articulation (100) dans laquelle s'applique le couple d'extension maximal.

17. Articulation artificielle selon l'une des revendications précédentes, **caractérisée en ce que** le moyen de transmission de force (8, 9) est réalisé sous forme de tige de poussée ou de tirant flexible.

18. Articulation artificielle selon l'une des revendications précédentes, **caractérisée par** une butée d'extension élastique (30) qui freine le mouvement d'extension dans une plage angulaire fixée avant d'atteindre l'extension maximale.
